(19) Europäisches Patentamt
European Patent Office
Office européen des brevets

(11) Veröffentlichungsnummer: **0 184 058**
**A2**

# (12) EUROPÄISCHE PATENTANMELDUNG

(21) Anmeldenummer: 85114568.0

(22) Anmeldetag: 16.11.85

(51) Int. Cl.[4]: C 07 D 209/28
A 61 K 31/40

(30) Priorität: 01.12.84 DE 3443993

(43) Veröffentlichungstag der Anmeldung: 11.06.86 Patentblatt 86/24

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Samaan, Samir, Dr.
Claudiusweg 9
D-5600 Wuppertal 1(DE)

## (54) Verfahren zur Herstellung von neuen Indolderivaten und deren Verwendung.

(57) Die neuen Indolderivate der Formel

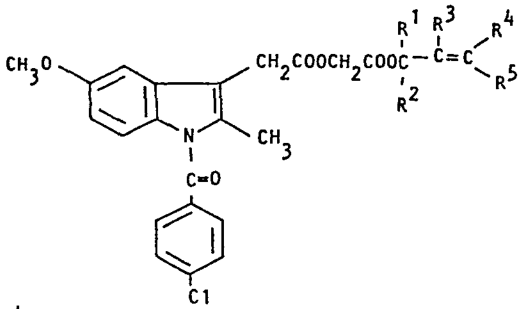

in der

$R^1$ und $R^2$ gleich oder verschieden sind und Wasserstoff oder Niederalkyl bedeuten,

$R^3$ Wasserstoff, Niederalkyl oder Halogen bedeuten,

$R^4$ und $R^5$ gleich oder verschieden sind und Wasserstoff, Niederalkyl oder gegebenenfalls substituiertes Aryl bedeuten, wobei $R^4$ und $R^5$ auch für Niederalkylen stehen können, und

wobei $R^2$ und $R^5$ durch eine Alkylenbrücke der Formel

$$-CH_2-CH_2-(CH_2)_n-$$

in der

n für die Zahl 0 oder 1 steht,

verbunden sein können, können durch Umsetzung eines Ammonium- oder Phosphoniumsalzes der entsprechenden Indolcarbonsäure mit einem Halogenessigsäure-allyl-ester hergestellt werden. Die neuen Verbindungen können zur Herstellung von Acemetacin verwendet werden. Die neuen Indolderivate haben eine pharmakologische Wirkung.

EP 0 184 058 A2

0184058

BAYER AKTIENGESELLSCHAFT 5090 Leverkusen, Bayerwerk

Konzernverwaltung RP Mn/by-c
Patentabteilung

# Verfahren zur Herstellung von neuen Indolderivaten und deren Verwendung

Die Erfindung betrifft neue Indolderivate, ein Verfahren zu ihrer Herstellung, ihre Verwendung zur Herstellung von Acemetacin und ihre Verwendung als Arzneimittel.

Die Herstellung der Indolderivate 1-(p-Chlorbenzyl)-5-methoxy-2-methylindol-3-acetoxyessigsäure-tetrahydropyranylester und des entsprechenden Furanylesters (der sogenannte Acemetacin-tetrahydropyranylester bzw. furanylester) ist aus der EP 00 87 655 bekannt. Ähnliche Verbindungen werden in der EP 00 88 252 und der EP 00 87 657 beschrieben.

Es wurden neue Indolderivate der Formel

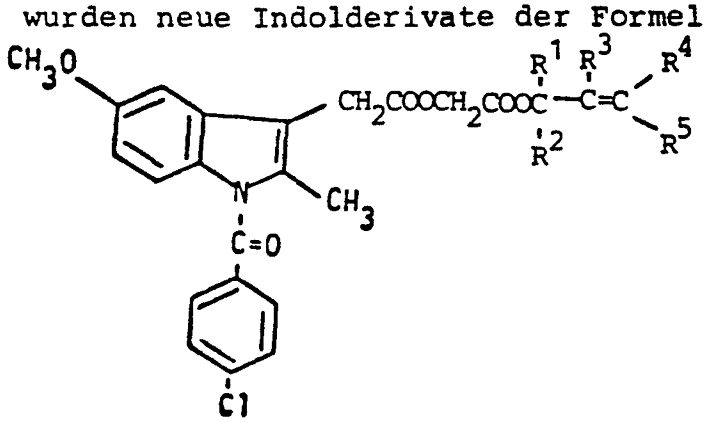

(I),

in der

$R^1$ und $R^2$ gleich oder verschieden sind und Wasserstoff oder Niederalkyl bedeuten,

$R^3$ Wasserstoff, Niederalkyl oder Halogen bedeuten,

$R^4$ und $R^5$ gleich oder verschieden sind und Wasserstoff, Niederalkyl oder gegebenenfalls substituiertes Aryl bedeuten, und wobei $R^4$ und $R^5$ auch für Niederalkenyl stehen können, und

wobei $R^2$ und $R^5$ durch eine Alkylenbrücke der Formel

$$-CH_2-CH_2(CH_2)_n$$

in der

n für die Zahl 0 oder 1 steht,

verbunden sein können, gefunden.

Die neuen Indolderivate sind 1-(p-Chlorbenzyl)-5-methoxy-2-methylindol-3-acetoxyessigsäureallylester (Acemetacin-allylester).

Sie können besonders vorteilhaft zur Herstellung von Acemetacin verwendet werden. Acemetacinallylester selbst haben eine bedeutende pharmakologische Wirkung.

Im Rahmen der vorliegenden Erfindung bedeutet Niederalkyl im allgemeinen einen geradkettigen oder verzweigten

Kohlenwasserstoffrest mit 1 bis etwa 6 Kohlenstoffatomen. Beispielsweise seien die folgenden Niederalkylreste genannt: Methyl, Ethyl, Propyl, Isopropyl, Butyl, Isobutyl, Pentyl, Isopentyl, Hexyl und Isohexyl. Besonders bevorzugte Niederalkylreste sind Methyl und Ethyl.

Im Rahmen der vorliegenden Erfindung bedeutet Niederalkenyl im allgemeinen einen ungesättigten, geradkettigen oder verzweigten Kohlenwasserstoffrest mit 2 bis etwa 6 Kohlenstoffatomen und mit bevorzugt einer Doppelbindung. Beispielsweise seien die folgenden Niederalkenylreste genannt: Vinyl, Allyl, Buten-(2)-yl und Penten-(2)-yl.

Im Rahmen der vorliegenden Erfindung bedeutet Halogen im allgemeinen Fluor, Chlor, Brom oder Iod, bevorzugt Chlor.

Im Rahmen der vorliegenden Erfindung bedeutet Aryl im allgemeinen einen aromatischen Kohlenwasserstoffrest mit 6 bis 12 Kohlenstoffatomen. Beispielsweise seien die folgenden Arylreste genannt: Phenyl, Biphenyl, Naphthyl und Benzyl. Bevorzugter Arylrest ist Phenyl.

Die Arylreste können gegebenenfalls substituiert sein. In diesem Fall ist der Arylrest im allgemeinen durch 1 bis 3, bevorzugt 1 oder 2, Reste substituiert.

Als mögliche Reste seien Halogene, wie Fluor, Chlor, Brom oder Iod, bevorzugt Chlor, oder Niederalkyl ($C_1$ bis etwa $C_6$), bevorzugt Methyl und Ethyl, genannt.

Falls die Reste $R^2$ und $R^5$ durch eine Alkylenbrücke verbunden sind, entstehen Cyclopentenyl- bzw. Cyclohexenylringe.

Indolderivate der Formel

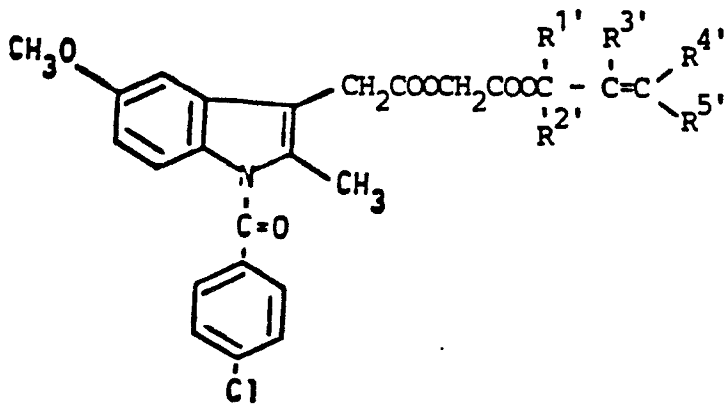

(II),

in der

$R^{1'}$ und $R^{2'}$ gleich oder verschieden sind und Wasserstoff, Methyl oder Ethyl bedeuten,

$R^{3'}$ Wasserstoff, Methyl, Ethyl oder Chlor bedeutet,

$R^{4'}$ und $R^{5'}$ gleich oder verschieden sind und Wasserstoff, Methyl, Ethyl, Phenyl, Benzyl, Tolyl oder 4-Chlor-phenyl bedeuten,

wobei $R^{2'}$ und $R^{5'}$ durch den Rest

$$-CH_2-CH_2-CH_2-$$

verbunden sein können, werden bevorzugt.

Im einzelnen seien die folgenden Acemetacinallylester genannt:

Acemetacin-allylester,
Acemetacin-methallylester,
Acemetacin-cinnamylester,
Acemetacin-2-chlor-allylester,
Acemetacin-crotylester,
Acemetacin-p-chlor-cinnamylester,
Acemetacin-cyclohexenylester und
Acemetacin-hexadienylester.

Im besonderen bevorzugt ist der Acemetacinallylester.

Es wurde auch ein Verfahren zur Herstellung der neuen Indolderivate gefunden, das dadurch gekennzeichnet ist, daß man Salze der Indolcarbonsäure der Formel

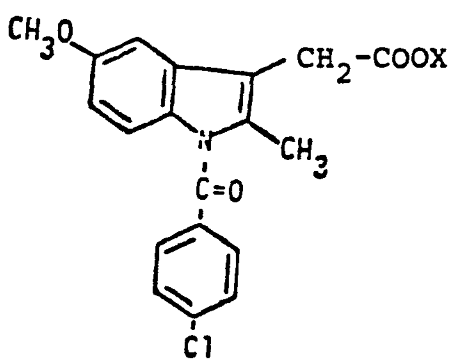

(III),

in der

X für Wasserstoff oder einen Ammonium- oder Phosphoniumrest steht,

mit einem Halogenessigsäureallylester der Formel

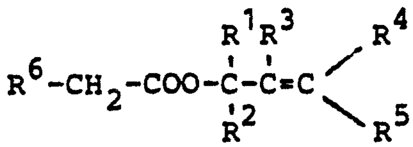

(IV),

in der

$R^1$ und $R^2$ gleich oder verschieden sind und Wasserstoff oder Niederalkyl bedeuten,

$R^3$ Wasserstoff, Niederalkyl oder Halogen bedeutet,

$R^4$ und $R^5$ gleich oder verschieden sind und Wasserstoff, Niederalkyl oder gegebenenfalls substituiertes Aryl bedeuten, wobei $R^4$ und $R^5$ auch für Niederalkenyl stehen können,

wobei $R^2$ und $R^5$ durch eine Alkylenbrücke der Formel

$$-CH_2-CH_2-(CH_2)_n-$$

in der

n für die Zahl 0 oder 1 steht,

verbunden sein können, und

$R^6$ für Halogen steht,

in Gegenwart von Lösungsmitteln im Temperaturbereich von -30 bis +110°C umsetzt.

Das erfindungsgemäße Verfahren kann durch die folgende Reaktionsgleichung erläutert werden:

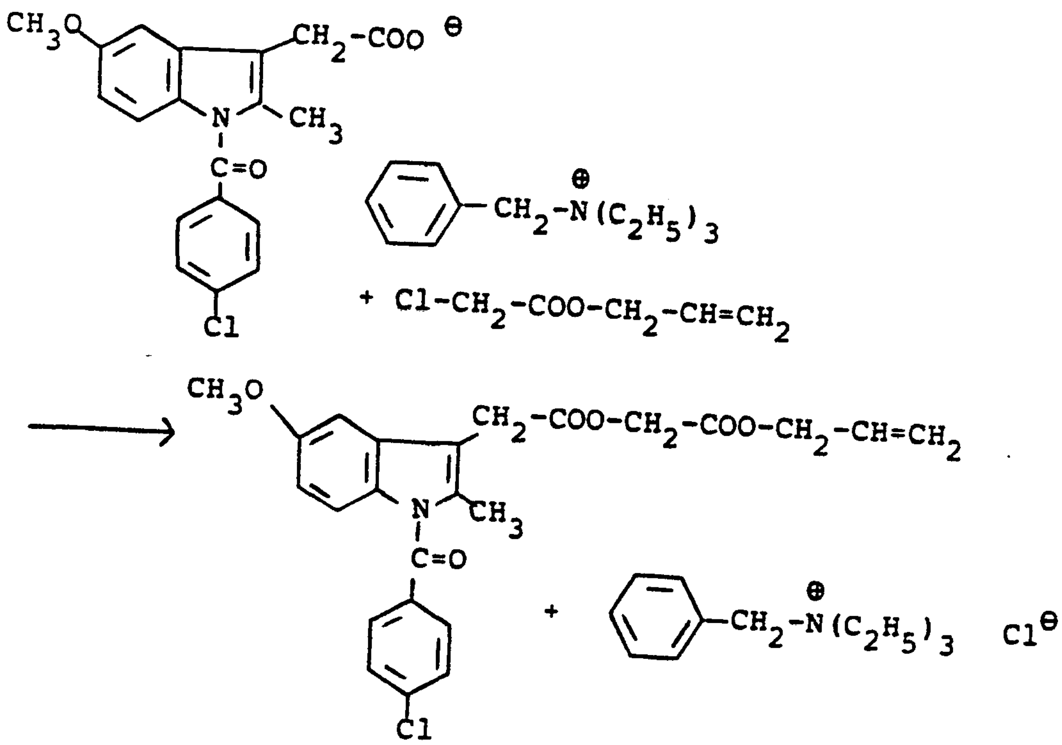

Die Ammonium- oder Phosphoniumsalze der Indolcarbonsäure für das erfindungsgemäße Verfahren sind an sich bekannt (Arzneimittelforschung 30, 1314 (1980)).

Als Ammonium- bzw. Phosphoniumreste seien Reste der Formel

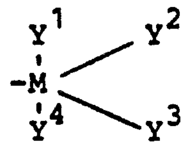

in der

M für Stickstoff oder Phosphor steht und

$Y^1$ bis $Y^4$ gleich oder verschieden sind und Wasserstoff, Alkyl, Cycloalkyl, Aryl bedeuten,

genannt.

Alkyl bedeutet hierbei erfindungsgemäß einen geradkettigen oder verzweigten Kohlenwasserstoffrest mit 1 bis etwa 12 Kohlenstoffatomen. Beispielsweise seien die folgenden Alkylreste genannt:

Methyl, Ethyl, Propyl, Isopropyl, Butyl, Isobutyl, Pentyl, Isopentyl, Hexyl und Isohexyl.

Aryl bedeutet hierbei erfindungsgemäß einen aromatischen Kohlenwasserstoffrest mit 6 bis 12 Kohlenstoffatomen. Beispielsweise seien Phenyl, Biphenyl, Benzyl und Naphthyl genannt.

Cycloalkyl bedeutet hierbei erfindungsgemäß einen cyclischen Kohlenwasserstoffrest mit 4 bis 8, bevorzugt 5 oder 6, Kohlenstoffatomen. Beispielsweise seien die folgenden Cycloalkylreste genannt: Cyclobutyl, Cyclopentyl, Cyclohexyl, Cycloheptyl und Cyclooctyl.

Besonders bevorzugt werden tertiäre oder quartäre Ammoniumsalze mit Methyl, Ethyl, Phenyl und Benzylresten.

Halogenessigsäureallylester für das erfindungsgemäße Verfahren sind an sich bekannt (J. Polymer. Sci. 3, 278 (1948), C.A, 2632 (1949)) und können beispielsweise durch Umsetzung von Halogenessigsäuren und Allylalkoholen oder aus Chloracetylchlorid bzw. Bromacetylbromid und Allylalkoholen in Gegenwart einer Base, z.B. Anilin hergestellt werden. Beispielsweise seien die folgenden Halogenessigsäureallylester genannt: Chloressigsäureallylester, Chloressigsäure-1-cinnamylester, Chloressigsäure-$\gamma$-methallylester, Bromessigsäure-allylester, Bromessigsäure-$\alpha$-methallylester, Chloressigsäure-cyclohexenylester, Chloressigsäure-$\alpha$-chlorallylester, Chloressigsäure-p-chlorcinnamylester, Chloressigsäure-hexadienylester und Chloressigsäure-crotylester.

Bevorzugte Halogenessigsäureallylester für das erfindungsgemäße Verfahren sind Chloressigsäure-allylester, Chloressigsäure-$\alpha$-methallylester, Chloressigsäure-cinnamylester.

Das erfindungsgemäße Verfahren wird im allgemeinen im Temperaturbereich von -30 bis +110°C, bevorzugt 0 und 50°C, durchgeführt.

Das erfindungsgemäße Verfahren wird im allgemeinen unter Normaldruck durchgeführt. Es ist aber auch möglich, das Verfahren bei einem Unter- oder Überdruck, beispielsweise im Druckbereich von 1 bis 6 bar, durchzuführen. Lösungsmittel für das erfindungsgemäße Verfahren sind im allgemeinen inerte organische Lösungsmittel. Bevorzugt werden polare, aprotische Lösungsmittel wie z.B. Chloroform, Dichlormethan, Dimethylformamid, Hexamethylphosphorsäure-triamid und Formamid.

In einer besonderen Ausführungsform des erfindungsgemäßen Verfahrens wird die Umsetzung in einer Zweiphasenreaktion durchgeführt. Hierbei kann in einem flüssig-flüssig Zweiphasensystem, wobei Wasser die zweite Phase darstellt, oder in einem fest-flüssig-Zweiphasensystem, mit der Indolcarbonsäure bzw. ihren Salzen als kristalline Phase gearbeitet werden. Als organische Lösungsmittel werden hierbei Toluol, Dichlormethan, Ethylacetat, Chloroform, Aceton und Methylisobutylketon besonders bevorzugt.

Bei der Durchführung des erfindungsgemäßen Verfahrens in einem Zweiphasensystem setzt man im allgemeinen einen Phasentransferkatalysator zu.

Phasentransferkatalysatoren für das erfindungsgemäße Verfahren können Verbindungen der Formel

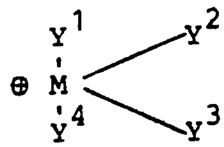 Anion$^{\ominus}$ (IV),

sein,
in der

$Y^1$ bis $Y^4$ die obengenannte Bedeutung haben und es sich bei dem Anion um einen anorganischen oder organischen Säurerest handelt.

Anione können beispielsweise Halogenide, bevorzugt Chloride und Bromide, Sulfate und Hydrogensulfate oder Acetate sein.

Bevorzugt seien die folgenden Phasentransferkatalysatoren genannt: Benzyl-triethylammoniumchlorid, Tetrabutylammoniumbromid, Tetrabutylammoniumhydrogensulfat, Methyl-trioctylammoniumchlorid, Tetrabutylphosphoniumbromid und Hexadecyl-tributylphosphoniumbromid.

Der Phasentransferkatalysator wird im allgemeinen in einer Menge von 0,05 bis 20 Mol-%, bevorzugt von 1 bis 10 Mol-%, bezogen auf die Indolcarbonsäure eingesetzt.

Bei Verwendung der freien Indolcarbonsäure wird das erfindungsgemäße Verfahren bevorzugt in Gegenwart von Basen durchgeführt werden. Als Basen seien Alkali (bevorzugt Natrium und Kalium)- und Erdalkali(bevorzugt Magnesium und Calcium)- Hydroxide und Carbonate genannt. Die Base wird im allgemeinen in einer Menge von

1 bis 20 Mol, bevorzugt 1,05 bis 10 Mol, bezogen auf 1 Mol Indolcarbonsäure eingesetzt.

Das erfindungsgemäße Verfahren kann beispielsweise wie folgt durchgeführt werden:
Die Reaktionskomponenten werden in dem gewählten Reaktionsmedium bei der erfindungsgemäßen Temperatur umgesetzt. Nach erfolgter Umsetzung wird in an sich bekannter Weise aufgearbeitet.

Die Aufarbeitung erfolgt im allgemeinen durch Trennung der wasserlöslichen Anteile durch Waschen mit Wasser, Filtrieren und Trocknen der organischen Phase und gegebenenfalls durch Chromatographieren an Siliciumdioxid.

Die erfindungsgemäßen Acemetacinallylester können zur Herstellung von Acemetacin verwendet werden. Acemetacin ist ein bekannter Wirkstoff mit anti-inflammatrischen Eigenschaften (Arzneimittelforschung 30, 1314 (1980)).

Die Herstellung des Acemetacins aus dem Acemetacinallylester ist dadurch gekennzeichnet, daß man den Acemetacinallylester in Gegenwart eines Phosphin-palladium-O-komplexes und eines Nucleophils im Temperaturbereich von 10 bis 150°C umsetzt.

Phosphin-palladium-O-komplexe für das erfindungsgemäße Verfahren sind beispielsweise Tetrakis-(triarylphosphin)-palladium-O-komplexe, beispielsweise die Verbindung

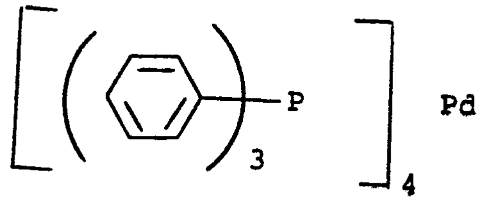

(VII),

in der der Phenylring durch weitere Reste, beispielsweise eine oder zwei Methyl- oder Ethylgruppen, substituiert sein kann.

Nucleophile für das erfindungsgemäße Verfahren sind beispielsweise primäre Amine, sekundäre Amine oder Anionen C-H-acider Verbindungen. Primäre und sekundäre Amine sind beispielsweise Verbindungen der Formel

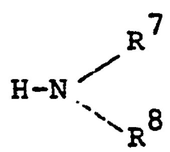

(VIII),

in der

$R^7$ und $R^8$ gleich oder verschieden sind und Niederalkyl, Aryl, Cycloalkyl und Niederalkenyl bedeuten, wobei $R^7$ und $R^8$ über eine Alkylenbrücke, die gegebenenfalls Stickstoff, Sauerstoff oder Schwefel als Heteroatome enthalten kann, verbunden sein können, und wobei $R^7$ oder $R^8$ auch Wasserstoff bedeuten können.

Die Niederalkyl-, Aryl-, Cycloalkyl- und Niederalkenylreste entsprechen dem oben angegebenen Bedeutungsumfang.

Falls $R^7$ und $R^8$ durch eine Brücke verbunden sind, entstehen 5- oder 6-gliedrige Ringe. Außer den Alkylengliedern können sie noch ein oder zwei, gleiche oder verschiedene Heteroatome enthalten.

C-H-acide Verbindungen sind beispielsweise Verbindungen der Formel

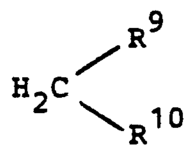 (IX),

in der

$R^9$ und $R^{10}$ gleich oder verschieden sind und Nitril, Acetyl, Benzoyl oder Alkoxy ($C_1$ bis $C_6$)-Carbonyl bedeuten und gegebenenfalls durch die Gruppen

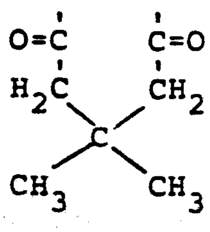

oder

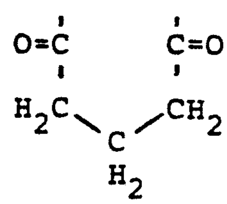

verbunden sein können.

Der Palladiumkomplex für das erfindungsgemäße Verfahren wird in katalytischer Menge eingesetzt. Im allgemeinen setzt man den Palladiumkomplex in einer Menge von 0,1 bis 10 Mol%, bezogen auf den Acemetacinallylester ein.

Bei Verwendung des Tetrakis-triphenylphosphin-palladiums arbeitet man in homogener Phase, wobei als Lösungsmittel inerte organische Lösungsmittel verwendet werden, bevorzugt sind Tetrahydrofuran, 1,4-Dioxan, Diethylether, Toluol, Chloroform und Methylenchlorid. Gegenstand der Erfindung ist auch die Verwendung von in situ erzeugten Phosphin-palladium-(O)-komplexen für diese Spaltung der allylischen Schutzgruppen. Hierbei wird ein üblicher Hydrierkatalysator aus Palladium auf Kohle (0,5 bis 5 %ig) verwendet und durch Zugabe der 4 bis 5-fachen molaren Mengen (auf Palladium bezogen) eines Triarylphosphins z.B. Triphenylphosphin der Palladium-(O)-komplex in situ erzeugt. Vorteil dieses Verfahrens ist die einfache Rückgewinnung des Edelmetalls durch einfache Filtration.

Die Herstellung des Acemetacins aus dem Acemetacinallylester kann beispielsweise wie folgt durchgeführt werden: Allylester, Palladium/Kohle und Triphenylphosphin werden in einem inerten Lösungsmittel, wie z.B. Toluol vorgelegt und tropfenweise mit Piperidin versetzt. Man rührt mehrere Stunden bei erhöhter Temperatur bis die Spaltung beendet ist. Es wird dann vom Katalysator abfiltriert und das Acemetacin aus dem Filtrat wie üblich isoliert.

Es ist besonders überraschend, daß sich Acemetacin erfindungsgemäß über die Acemetacinallylester in hohen Ausbeuten und hohen Reinheiten herstellen läßt, weil

weder sauer noch alkalisch hydrolysiert wird, wie dies üblicherweise bei der Abspaltung von Schutzgruppen gearbeitet wird (vgl. z.B. T.W. Green, Protective Groups in Organic Synthesis, Wiley, New York, 1981). Nur der Allylester wird unter diesen Bedingungen gespalten, während die aktivierte Methylesterstruktur unberührt bleibt.

Die erfindungsgemäßen Acemetacinallylester haben eine hervorragende pharmakologische Wirkung. Bevorzugt ist die Verwendung der erfindungsgemäßen Acemetacinallylester bei der Bekämpfung von entzündungsbedingten Erkrankungen, bei der Bekämpfung von Erkrankungen des rheumatischen Formenkreises und die Verwendung als Antiphlogistika.

So wurden an dem bekannten pharmakologischen Entzündungsmodell, dem Kaolinödem der Rattenpfoten (Z.ges. exp.Med. 131, 407 (1959)) Ergebnisse erzielt, die denen der in der Pharmazie verwendeten Entzündungshemmern entsprechen und teilweise sogar übertreffen. Auch in der Steigerung der Sulfhydrylgruppenaktivität von Serumproteinen, welche ein Maß für die Wirksamkeit von Antiphlogistika darstellt (Biochem. Pharmacol. 16, 115 (1967)), waren die Mehrzahl der erfindungsgemäßen Verbindungen der als Vergleichssubstanz verwendeten Flufenaminsäure deutlich überlegen.

Zur vorliegenden Erfindung gehören ebenfalls pharmazeutische Zubereitungen, die neben nicht-toxischen, inerten, pharmazeutisch geeigneten Trägerstoffen einen oder mehrere

erfindungsgemäße Wirkstoffe enthalten oder die aus einem oder mehreren erfindungsgemäßen Wirkstoffen bestehen sowie Verfahren zur Herstellung dieser Zubereitungen.

Unter nicht-toxischen, inerten, pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure, (b) Bindemittel, z.B. Carboxymethylzellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quartäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofette und höhere Ester (z.B. $C_{14}$-Alkohol mit $C_{16}$-Fettsäure) und Wachse oder Gemische dieser Stoffe.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylencarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Zellulose, Aluminiummetahydroxid, Bentonit, Agar-Agar, und Tragant oder Gemische dieser Stoffe enthalten.

Die genannten Formulierungsformen können auch Färbemittel, Konservierungsstoffe sowie geruchs- und geschmacks-verbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5 Gew.-%, vorzugsweise von etwa 0,5 bis 95 Gew.-%, der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten

Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/ oder rectal, vorzugsweise oral, appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,1 bis etwa 200, vorzugsweise 0,5 bis 10 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von 0,1 bis 2,0 mg/kg Körpergewicht. Es kann jedoch erforderlich sein, von den genannten Dosierungen abzuweichen und zwar in Abhängigkeit von der Art und dem Körpergewicht des zu behandelnden Objektes, der Art und der Schwere der Erkrankung.

Beispiel 1

Allyl-1(4-chlorbenzoyl)-5-methoxy-2-methyl-3-indol-acetoxy-acetat (Acemetacin-allylester)

25 g Indometacin, 10 g Kaliumcarbonat und 1,0 g Benzyltriethylammoniumchlorid in 140 ml Aceton werden 30 Minuten bei 50 bis 60°C gerührt. Anschließend werden 10,1 g Chloressigsäure-allylester in 5 Minuten zugetropft. Man rührt bei 40°C solange nach bis die Reaktion beendet ist (DC-Kontrolle, ca. 3 Stunden). Es wird warm abfiltriert, das Filtrat im Vakuum eingedampft und der Rückstand in Ether aufgenommen (ca. 90 ml) und mit Petrolether (40 bis 60°C) versetzt (ca. 30 ml). Man läßt über Nacht bei -10° stehen, saugt ab und trocknet im Vakuum.

Ausbeute: 28,0 g ≙ 87,7 % d. Th.; Schmp. 59-61°C.

| | | | | | |
|---|---|---|---|---|---|
| $C_{24}H_{22}ClNO_6$ (455,5) | Ber. C: | 63,2 | H: 4,8 | N: 3,1 | Cl: 7,8 |
| | Gef. | 63,1 | 4,9 | 3,1 | 7,6 |

Beispiel 2

Cinnamyl-1-(4-chlorbenzoyl)-5-methoxy-2-methyl-3-indol-acetoxy-acetat (Acemetacin-cinnamylester)

10 g Indometacin, 4 g Kaliumcarbonat, 0,3 g Benzyltriethylammoniumchlorid und 50 ml Aceton werden 30 Minuten

bei 50-60° gerührt. Nach Zugabe von 6,32 g Chloressigsäure-cinnamylester rührt man bei 40° 3 bis 4 Stunden nach (DC-Kontrolle). Nach Filtration und Abdestillieren des Lösungsmittels wird der Rückstand zuerst aus Ether-Petrolether und dann aus Aceton-Wasser umkristallisiert.

Ausbeute: 8,6 g ≙ 58 % d.Th.; Schmp. 89-91°C

| | | | | | |
|---|---|---|---|---|---|
| $C_{30}H_{27}ClNO_6$ (532,0): | Ber. | C 67,7 | H 4,9 | N 2,6 | Cl 6,6 |
| | Gef. | 67,8 | 4,9 | 2,6 | 6,7 |

Beispiel 3

Methallyl-1-(4-chlorbenzoyl)-5-methoxy-2-methyl-3-indolacetoxy-acetat (Acemetacin-methallylester)

10 g Indometacin, 4,0 g Kaliumcarbonat, 0,4 g Benzyltriethylammoniumchlorid und 60 ml Aceton werden 30 Minuten bei 56°C gerührt. Nach Zugabe von Chloressigsäuremethallylester rührt man 3 Stunden bei 40°C nach (DC-Kontrolle). Es wird abfiltriert und im Vakuum eingedampft. Der Rückstand wird in Toluol warm gelöst und vom ungelösten abfiltriert. Nach Abkühlen wird der Ester mit Petrolether (40-60°C) ausgefällt.

Ausbeute: 11 g ≙ 84 % d. Th.; Schmp. 74-76°C

| | | | | | |
|---|---|---|---|---|---|
| $C_{25}H_{24}ClNO_6$ (469,5): | Ber. | C 63,9 | H 5,1 | N 3,0 | Cl 7,5 |
| | Gef. | 64,0 | 5,2 | 3,0 | 7,5 |

Beispiel 4

Acemetacin

0,03 Mol Acemetacin-allylester in 50 ml Tetrahydrofuran werden unter Stickstoff mit 0,003 Mol Tetrakis-triphenylphosphin-palladium-(O) versetzt. Bei 20-25°C tropft man unter Rühren 0,3 Mol Piperidin zu und rührt anschließend 2 Stunden bei Raumtemperatur nach (DC-Kontrolle). Unter Kühlung tropft man 200 ml halbkonzentrierte Salzsäure zu, trennt die Phasen, extrahiert die Wasserphase 2 mal mit Dichlormethan und dampft die vereinigten organischen Phasen im Vakuum ein. Der Rückstand wird aus Toluol umkristallisiert.

Ausbeute: 86-91 % d. Th.; Schmp. 150°C

Beispiele 5 und 6

Bei analoger Arbeitsweise zu Beispiel 4 erhält man aus

| | | |
|---|---|---|
| Acemetacin-cinnamylester | 81 % d.Th. | und aus |
| Acemetacin-methallylester | 86 % d. Th. | Acemetacin. |

Beispiel 7

Acemetacin

Unter Stickstoff werden 31,9 g Acemetacin-allylester, 100 ml

Toluol, 2,3 g Triphenylphosphin und 0,75 g Palladium-Kohle (5 %ig) vorgelegt. Unter Rühren tropft man 11,9 g Piperidin zu und rührt anschließend 3 Stunden bei 90°C nach. Es wird heiß abfiltriert, und der Katalysator mit heißem Toluol nachgewaschen. Das Filtrat wird eingedampft, der Rückstand in Dichlormethan (ca. 300 ml) aufgenommen, mit halbkonzentrierter Salzsäure (100 ml) und dann mit Wasser gewaschen. Nach Entfernung des Lösungsmittels im Vakuum wird der Rückstand aus Toluol umkristallisiert.

Ausbeute: 27,3 g ≙ 94 % d. Th.; Schmp. 150°

Beispiele 8 und 9

Bei analoger Arbeitsweise zu Beispiel 7 erhält man aus

| | | |
|---|---|---|
| Acemetacin-cinnamylester | 89 % d. Th. | und aus |
| Acemetacin-methallylester | 84 % d. Th. | Acemetacin. |

0184058

Patentansprüche

1. Indolderivate der Formel

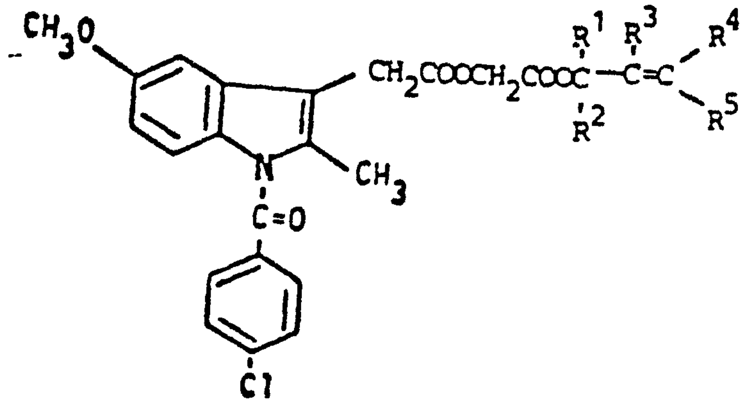

in der

$R^1$ und $R^2$ gleich oder verschieden sind und Wasserstoff oder Niederalkyl bedeuten,

$R^3$ Wasserstoff, Niederalkyl oder Halogen bedeuten,

$R^4$ und $R^5$ gleich oder verschieden sind und Wasserstoff, Niederalkyl oder gegebenenfalls substituiertes Aryl bedeuten, wobei $R^4$ und $R^5$ auch für Niederalkenyl stehen können, und

wobei $R^2$ und $R^5$ durch eine Alkylenbrücke der Formel

$$-CH_2-CH_2-(CH_2)_n$$

in der

n für die Zahl 0 oder 1 steht

verbunden sein können.

2. Indolderivate nach Anspruch 1 der Formel

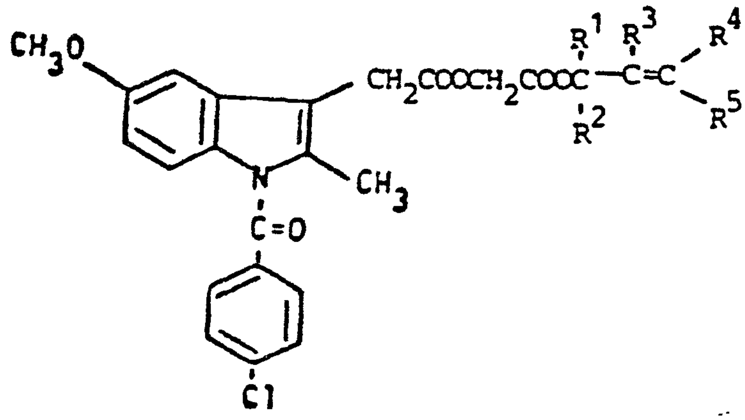

in der

$R^{1'}$ und $R^{2'}$ gleich oder verschieden sind und Wasserstoff, Methyl oder Ethyl bedeuten,

$R^{3'}$ Wasserstoff, Methyl, Ethyl oder Chlor bedeutet,

$R^{4'}$ und $R^{5'}$ gleich oder verschieden sind und Wasserstoff, Methyl, Ethyl, Phenyl, Benzyl, Tolyl oder 4-Chlor-phenyl bedeuten,

wobei $R^{2'}$ und $R^{5'}$ durch den Rest

$$-CH_2-CH_2-CH_2-$$

verbunden sein können.

3. Verfahren zur Herstellung von Indolderivaten, dadurch gekennzeichnet, daß man Salze der Indolcarbonsäure der Formel

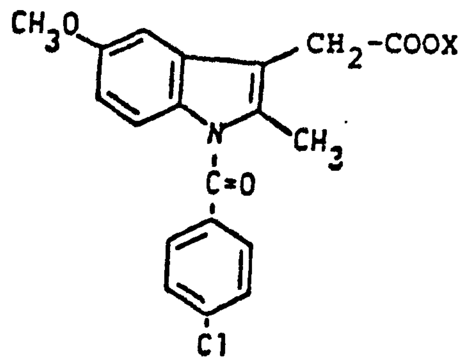

in der

X für Wasserstoff oder ein Ammonium- oder Phosphoniumrest steht

mit einem Halogenessigsäurealkylester der Formel

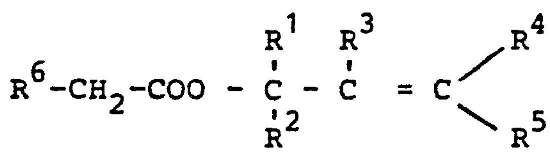

in der

$R^1$ und $R^2$ gleich oder verschieden sind und Wasserstoff oder Niederalkyl bedeuten,

$R^3$ Wasserstoff, Niederalkyl oder Halogen bedeutet,

$R^4$ und $R^5$ gleich oder verschieden sind und Wasserstoff, Niederalkyl oder gegebenenfalls substi-

tuiertes Aryl bedeuten, wobei $R^4$ oder $R^5$ auch für Niederalkenyl stehen können und

wobei $R^2$ und $R^5$ durch eine Alkylenbrücke der Formel

$$-CH_2-CH_2-(CH_2)_n-$$

in der

n für die Zahl 0 oder 1 steht,

verbunden sein können, und

$R^6$ für Halogen steht,

in Gegenwart von Lösungsmitteln im Temperaturbereich von -30 bis +110°C umsetzt.

4. Verfahren nach Anspruch 3, dadurch gekennzeichnet, daß man die Umsetzung in einem Gemisch aus organischem Lösungsmittel und Wasser durchführt.

5. Verfahren nach den Ansprüchen 3 und 4, dadurch gekennzeichnet, daß es in Gegenwart einer Base durchgeführt wird.

6. Verwendung von Indolderivaten der Formel

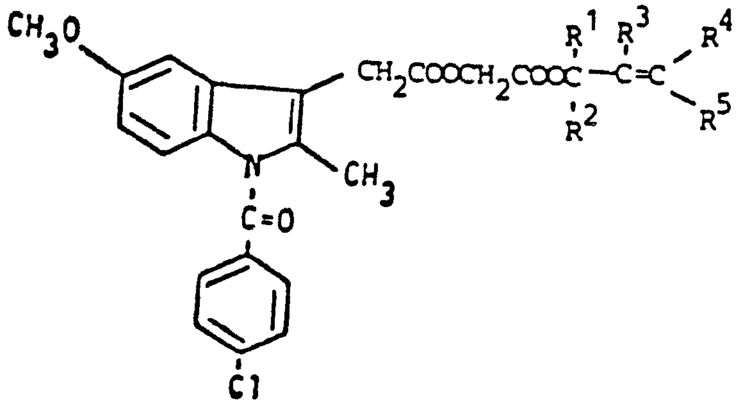

in der

$R^1$ und $R^2$ gleich oder verschieden sind und Wasserstoff oder Niederalkyl bedeuten,

$R^3$ Wasserstoff, Niederalkyl oder Halogen bedeuten,

$R^4$ und $R^5$ gleich oder verschieden sind und Wasserstoff, Niederalkyl oder gegebenenfalls substituiertes Aryl bedeuten, wobei $R^4$ und $R^5$ auch für Niederalkenyl stehen können, und

wobei $R^2$ und $R^5$ durch eine Alkylenbrücke der Formel

$-CH_2-CH_2-(CH_2)_n$

in der

n für die Zahl 0 oder 1 steht,

verbunden sein können,

zur Herstellung von Acemetacin.

7. Verwendung nach Anspruch 6, dadurch gekennzeichnet, daß man das Indolderivat in Gegenwart eines Phosphin-palladium-0-komplexes und eines Nucleophils im Temperaturbereich von 10 bis 150°C umsetzt.

8. Arzneimittel, enthaltend ein Indolderivat der Formel

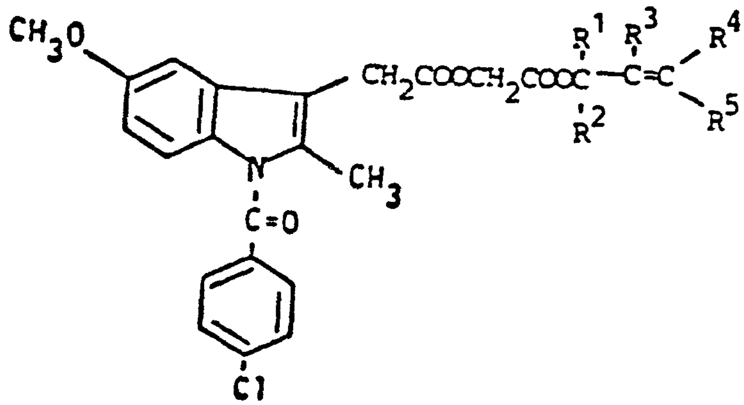

in der

$R^1$ und $R^2$ gleich oder verschieden sind und Wasserstoff oder Niederalkyl bedeuten,

$R^3$ Wasserstoff, Niederalkyl oder Halogen bedeuten,

$R^4$ und $R^5$ gleich oder verschieden sind und Wasserstoff, Niederalkyl oder gegebenenfalls substituiertes Aryl bedeuten, wobei $R^4$ und $R^5$ auch für Niederalkenyl stehen können, und

wobei $R^2$ und $R^5$ durch eine Alkylenbrücke der Formel

$$-CH_2-CH_2-(CH_2)_n$$

in der

n für die Zahl 0 oder 1 steht

verbunden sein können.

9. Verwendung von Indolderivaten der Formel

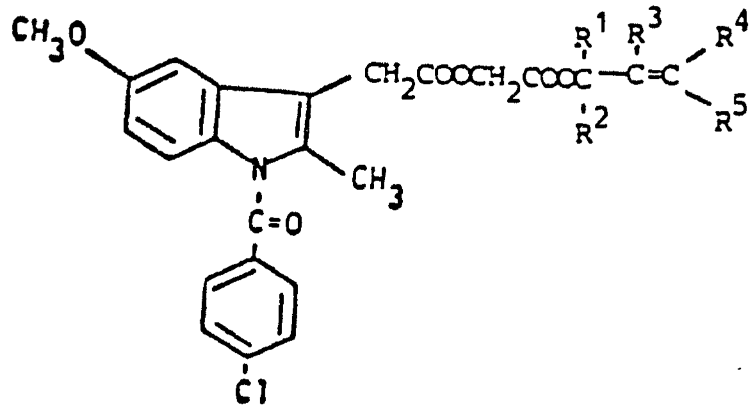

in der

$R^1$ und $R^2$ gleich oder verschieden sind und Wasserstoff oder Niederalkyl bedeuten,

$R^3$ Wasserstoff, Niederalkyl oder Halogen bedeuten,

$R^4$ und $R^5$ gleich oder verschieden sind und Wasserstoff, Niederalkyl oder gegebenenfalls substituiertes Aryl bedeuten, wobei $R^4$ und $R^5$ auch für Niederalkenyl stehen können, und

wobei $R^2$ und $R^5$ durch eine Alkylenbrücke der Formel

$$-CH_2-CH_2-(CH_2)_n$$

in der

n für die Zahl 0 oder 1 steht,

verbunden sein können, zur Herstellung von Arzneimitteln für die Bekämpfung von entzündungsbedingten Erkrankungen.

10. Verwendung von Indolderviaten der Formel

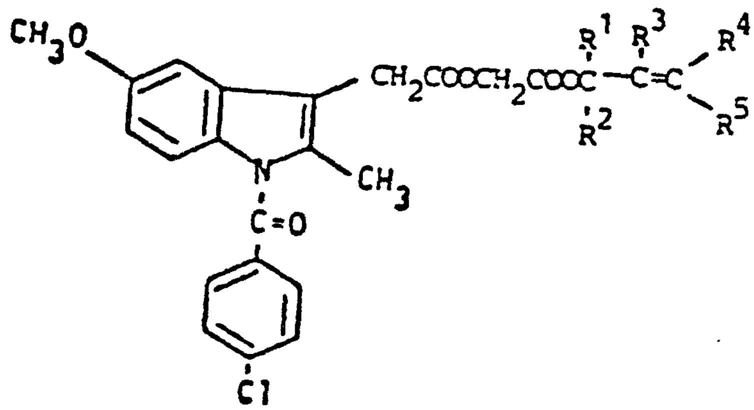

in der

$R^1$ und $R^2$ gleich oder verschieden sind und Wasserstoff oder Niederalkyl bedeuten,

$R^3$ Wasserstoff, Niederalkyl oder Halogen bedeuten,

$R^4$ und $R^5$ gleich oder verschieden sind und Wasserstoff, Niederalkyl oder gegebenenfalls substituiertes Aryl bedeuten, wobei $R^4$ und $R^5$ auch für Niederalkenyl stehen können, und

wobei $R^2$ und $R^5$ durch eine Alkylenbrücke der Formel

$$-CH_2-CH_2-(CH_2)_n$$

in der

n für die Zahl 0 oder 1 steht

verbunden sein können,

zur Herstellung von Arzneimitteln für die Bekämpfung von Erkrankungen des rheumatischen Formenkreises.

11. Verfahren zur Herstellung von Arzneimitteln, dadurch gekennzeichnet, daß man Indolderivate der Formel

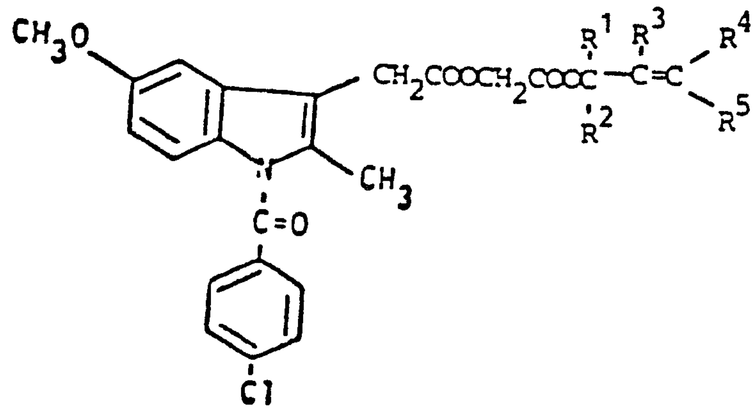

in der

$R^1$ und $R^2$ gleich oder verschieden sind und Wasserstoff oder Niederalkyl bedeuten,

$R^3$ Wasserstoff, Niederalkyl oder Halogen bedeuten,

$R^4$ und $R^5$ gleich oder verschieden sind und Wasserstoff, Niederalkyl oder gegebenenfalls substituiertes Aryl bedeuten, wobei $R^4$ und $R^5$ auch für Niederalkenyl stehen können, und

wobei $R^2$ und $R^5$ durch eine Alkylenbrücke der Formel

$$-CH_2-CH_2-(CH_2)_n$$

in der

n für die Zahl 0 oder 1 steht

verbunden sein können,

gegebenenfalls unter Verwendung von Hilfsstoffen und Trägerstoffen in eine geeignete Applikationsform überführt.